(19) Europäisches Patentamt
European Patent Office
Office européen des brevets

(11) Publication number: **0 124 377 B1**

# (12) EUROPEAN PATENT SPECIFICATION

(45) Date of publication of patent specification: **25.09.91**

(51) Int. Cl.[5]: **C07D 307/94**, C07D 491/107, B41M 5/145, B41M 5/26, //(C07D491/107,307:00,221:00), (C07D491/107,307:00,241:00)

(21) Application number: **84302885.3**

(22) Date of filing: **30.04.84**

The file contains technical information submitted after the application was filed and not included in this specification

## (54) Chromogenic compounds.

(30) Priority: **28.04.83 JP 74102/83**

(43) Date of publication of application:
**07.11.84 Bulletin 84/45**

(45) Publication of the grant of the patent:
**25.09.91 Bulletin 91/39**

(84) Designated Contracting States:
**AT BE CH DE FR GB IT LI NL SE**

(56) References cited:
**CH-A- 539 033**
**SU-A- 615 112**
**US-A- 3 344 189**
**US-A- 3 853 869**

**CHEMICAL ABSTRACTS, vol. 89, 1978, page 76, no. 148275b, Columbus, Ohio, US;**

(73) Proprietor: **YAMAMOTO KAGAKU GOSEI CO., LTD.**
**1 -43, Yuge-cho minami**
**Yao - Shi Ohsaka - Pref.(JP)**

(72) Inventor: **Ikegami, Seishi**
**100-20 Tennojiya**
**Yao-shi Osaka Pref.(JP)**
Inventor: **Amako, Takamichi**
**3-32-1 Shiki-cho Minami**
**Yao-shi Osaka Pref.(JP)**

(74) Representative: **Norris, Richard John et al**
**The Wiggins Teape Group Limited Group Patents Department Butler's Court**
**Beaconsfield Buckinghamshire HP9 1RT(GB)**

**EP 0 124 377 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to chromogenic compounds, and in particular to novel fluorene-spiro-lactone compounds, a process for their manufacture and record materials comprising the compounds.

More particularly, this invention relates to novel chromogenic fluorene-spiro-lactone compounds which can give intense colours when they are contacted with an electron accepting co-reactant and are suitable for use in pressure sensitive or heat-sensitive (or thermal) mark-forming record systems.

Pressure sensitive copying systems provide a mark-forming system disposed on and/or within a sheet support material in which a colour former, a co-reactant and a liquid solvent in which either or both the colour former and co-reactant are soluble. The liquid solvent is present in a form in which it is isolated from either or both the colour former and co-reactant by a barrier which is pressure rupturable to permit local reactive contact between the solvent, colour former and co-reactant to form a coloured mark by reaction of the colour former and co-reactant. The pressure rupturable barrier is most commonly formed by microcapsules containing the liquid solvent. Typically the encapsulated solvent includes either the colour former or co-reactant in solution and, most usually, the solution is of the colour former. In the present invention the microencapsulation technique used in preparing pressure sensitive copying systems can be those known in the art, particularly those in current use. Such systems can be based on coacervation of natural or synthetic colloids or in situ polymerisation of monomers, whether or not by coacervation. Among the microencapsulation techniques which can be used in this invention are those described in US Patent Specifications Nos. 2800457, 3041289, 3533958, 3755190, 4001140 and 4100103 and UK Patent Specification Nos. 1507739 and 2073132, to which reference can be made for process information.

Suitable solvents for preparing colour former containing microcapsules are those used in the art and include aromatic hydrocarbon solvents such as alkylated benzenes, naphthalenes and biphenyls; benzylated benzenes; and partially hydrogenated terphenyls, ester solvents such as phthalate and benzoate esters, long chain alcohols, phosphate ester solvents, and long chain alcohols, phosphate ester solvents, and long chain aliphatic hydrocarbons such as kerosenes ($C_9$ to $C_{14}$ alkanes) which are not themselves especially good solvents but are useful as diluents with other solvents.

The electron accepting or acidic co-reactant is usually either an inorganic acid reacting material or synthetic mineral especially activated clays e.g. attapulgite, bentonite and monotmorillonite, acid washed reactive clays such as silton clay as disclosed in US Specifications 3622364 and 3753761, materials such as silica gel, talc, feldspar, magnesium trisilicate, pyrophyllite, zinc sulphate, zinc sulphide, calcium sulphate, calcium citrate, calcium phosphate, calcium fluoride and barium sulphate, or an organic acid reacting material including phenols and diphenols as are disclosed in US Specification 3539375; aromatic carboxyl acids such as salicylic acid and derivatives thereof, metal salts of aromatic carboxylic acids, especially zinc salts, such as are disclosed in US Specification 4022936 and acidic organic polymers such as phenol-formaldehyde polymers such as disclosed in US Specification 3672935 and oil-soluble metal, especially zinc, salts of phenol-formaldehyde polymers as disclosed in US Specification 3732120.

Thermal record systems provide a mark-forming system disposed on a sheet support material in which a colour former and a co-reactant are dispersed. Generally, in thermal record systems the colour former and co-reactant do not react at ambient temperature because both are solid. The colour forming reaction takes place when the record material is heated usually to make one or both of them fluid, usually liquid. A binder is included to ensure adhesion to the substrate and uniform distribution of the solid reagents. Commonly other components are included e.g. fusible waxes to modify the fusion temperature, pigments either to colour or opacify the paper or to ensure rapid absorption of the coloured products and to reduce abrasion of and 'picking' of the paper by the thermal imaging element.

Pressure and heat sensitive material of the type described above have achieved widespread acceptance in many fields of business. The advent of wide scale use of computers and automated handling of information have led to an increased use of mechanised optical reading of documents. Optical character recognition (OCR) devices have been developed which are capable of reading pages of text printed or typed in a format that the OCR device is programmed to read. Typically, such devices read in the near infrared and although original print and type have absorptions in the near infrared which can be OCR detected, this has not been true of copies produced using pressure sensitive record material and even the originals of imaged heat sensitive record material as described above, which have, accordingly, not been readable by such OCR devices. Chromogenic materials having coloured forms with absorption bands in the near infrared are disclosed in U.S. Patent Specifications Nos. 4020056, 4022771, 4026883, 4107428 and 4119776.

The present invention is based on the discovery of a novel class of fluorene-spiro-lactone, and especially fluorene-spiro-phthalide, compounds which are chromogenic and have coloured forms with

absorption bands in the near infrared. Dyestuffs containing a fluorene ring as a part of their structure and methods of making them are disclosed in U.S. Patent Specifications Nos. 3344189 and 3413071 and German Offenlegungsschrift No. 2145027.

The present invention accordingly provides a compound of the general formula:

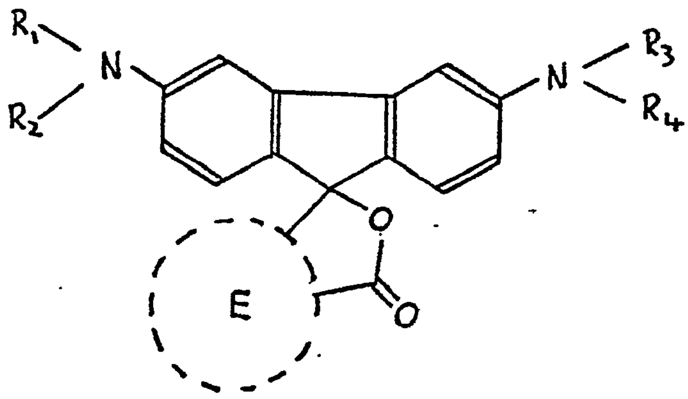

(I)

where E is a group of one of the formulae:

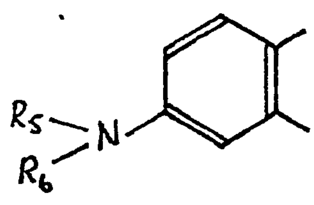

(II),

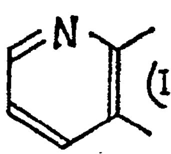

(III) or

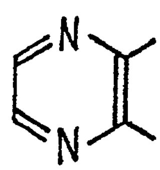

(IV)

$R_1$, $R_2$, $R_3$, $R_4$, $R_5$ and $R_6$ are each independently a hydrogen atom, a $C_1$ to $C_{12}$ alkyl group, a cycloalkyl group or a phenyl or benzyl group optionally substituted with one or more lower alkyl groups or $R_1$ and $R_2$, $R_3$ and $R_4$ and/or $R_5$ and $R_6$, together with the nitrogen atom to which they are attached represent a 5 or 6 membered heterocyclic ring group.

The invention includes a method of making a compound of the general formula (I) which comprises diazotizing a compound of the general formula:

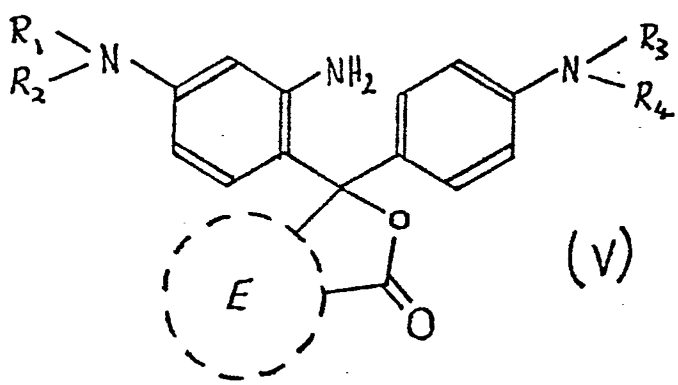

(V)

where $R_1$, $R_2$, $R_3$, $R_4$ and E are as defined above, and thereafter effecting ring closure.

In formula I where any of $R_1$, $R_2$, $R_3$, $R_4$, $R_5$ and $R_6$ are alkyl groups it can be straight or branched chain, it is generally preferred that it is a $C_1$ to $C_8$ and particularly $C_1$ to $C_4$ alkyl groups, the colour efficiency is greater with smaller alkyl groups as the compound has a lower molecular weight, however, if enhanced solubility in hydrocarbon sovents is a desired property, then it may be advantageous that one or more of the alkyl groups is a $C_6$ to $C_{12}$, especially $C_8$, alkyl group. Where any of these groups is a cycloalkyl group it will usually be a 5- or 6- membered group especially a cyclohexyl group. Where any of $R_1$ and $R_2$, $R_3$ and $R_4$ and $R_5$ and $R_6$, together with the nitrogen atom to which they are attached represent

a 5- or 6- membered heterocyclic ring group, this group will typically be a pyrrolidino or a piperidino e.g. pipecolino, group. When any of $R_1$, $R_2$, $R_3$, $R_4$, $R_5$ and $R_6$ is a lower alkyl substituted phenyl or benzyl group the or each lower alkyl group is typically a $C_1$ to $C_5$, preferably a $C_1$ to $C_3$, alkyl group.

Within the general formula (I) above 3,6-diaminofluorene-9-spiro-3'(6'-amino)phthalides of the general formula:

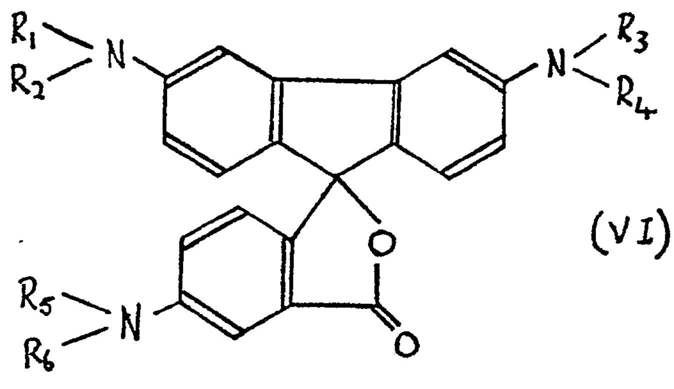

(VI)

where each of $R_1$, $R_2$, $R_3$, $R_4$, $R_5$ and $R_6$ is independently a $C_1$ to $C_4$ alkyl group, are particularly preferred.

The compounds of this invention can be made by the diazotization of the corresponding precursor amino substituted phthalide, furopyridine, or furopyrazine, followed by a ring closing condensation. The diazotization reaction is typically carried out in solution in sulphuric acid, sodium nitrite and sulphuric acid is a convenient diazotization agent, at a temperature of from -5 to 10 °C. We have found that raising the temperature of the sulphuric acid solution of the diazo intermediate to from 10 to 100 °C leads to a ring closing reaction. The ring closing reaction typically takes several hours, and may take some tens of hours, to go substantially to completion. Preferably the ring closing reaction is carried out at from 20 to 90 °C. The speed of reaction and the yield of the desired product can be enhanced by adding a catalytic amount of copper, either as elemental copper, especially copper powder, or in a copper compound, to the reaction mixture before the ring closing reaction. The compounds of the invention can be recovered from the reaction mixture by conventional methods, for example quenching into ice water, neutralising with alkai such as caustic soda, filtering off the precipitate and purifying it for example by recrystallization from a suitable organic solvent e.g. toluene. Typically, using this route, we have obtained the compounds of the invention as substantially colourless crystals.

The compounds of this invention are useful as chromogenic materials in pressure and/or heat sensitive record material and specifically record material in which the images can be detected and, given appropriate character formation, recognized by an optical character recognition (OCR) device sensitive in the near infrared. In their uncoloured form the compounds of the invention have substantially no absorption in the near infrared but when brought into reactive contact with a suitable acidic material such as is conventionally used in pressure and/or heat sensitive record material, they form intense visible colours which additionally have strong absorptions in the near infrared. The image also has excellent light fastness. The visible colours given by compounds of the formula (I) where E is a group of the formula (II) are typically greens and where E is a group of the formula (III) or (IV) the visible colours are redder and are typically browns.

Thus, in a further aspect the present invention provides record material incorporating, as a colour former at least one chromogenic fluorene-spiro-lactone compound of the invention. The invention includes a manifold set of pressure sensitive record material which comprises an upper sheet carrying on the lower surface thereof a coating of a microencapsulated solution of a chromogenic composition including at least one compound of this invention (the CB sheet), a lower sheet carrying on the upper surface thereof a coating comprising an electron accepting (acidic) co-reactant (the CF sheet) and, optionally, one or more intermediate sheets each of which has on its upper surface a coating comprising an electron accepting (acidic) co-reactant and on its lower surface a coating comprising a microencapsulated solution of a chromogenic composition, including at least one compound of this invention (CFB sheet(s)).

The invention includes heat sensitive record material which comprises a sheet, especially a paper sheet, carrying a coating comprising finely divided solid particles of a chromogenic compound of the invention and finely divided solid particles of an acidic co-reactant dispersed in a thermographically acceptable binder. The coating may include a fusible wax or other adjunct to reduce the effective reaction temperature and/or increase the image to background contrast, and a pigment to improve the appearance of

the record material and/or reduce 'picking' by the thermal imaging element.

As is noted above, the compounds of this invention generally have blue to green coloured forms. Most commonly, it is desired to have record material which gives a perceived black image. It will be appreciated by those skilled in the art that combining the compounds of the invention with chromogenic materials having coloured forms of a different colour can give perceived black images which will also be "coloured" in the near infrared by the presence of the compound of the invention.

The invention further provides a method of making an image comprising a mark having a substantial absorption in the visible spectrum, which mark is also detectable by optical character recognition apparatus sensitive to detect images having substantiasl absorption in the near infrared, which comprises forming an image on pressure sensitive or heat sensitive record material of the invention whereby to form an image including a coloured mark formed by reaction of a compound of the formula (I) with an electron accepting (acidic) co-reactant.

The following Examples illustrate the invention. All parts and percentages are by weight unless otherwise stated.

Example 1

3,6-bis(dimethylamino)fluorene-9-spiro-3'-(6'-dimethylamino)phthalide

8.6 parts of 3-(2-amino-4-dimethylaminophenyl)-3-(4-dimethylamino-phenyl)-6-dimethylaminophthalide were dissolved in a mixture of 37 parts of concentrated sulphuric acid and 12 parts of water and coooled to 0° C. To this was added dropwise over a period of about 30 min. a solution of 1.4 parts of sodium nitrite in 37 parts of concentrated sulphuric acid at 0 to 5° C, and the reaction was continued at that temperature for 2 hours to complete azotization. 0.2 g of copper powder was added and then the temperature of the reaction mixture was slowly raised to 80° C and the reaction mixture was stirred for about 20 hours at 80 to 90° C to complete ring closure. The reaction mixture was cooled, poured into 500 parts of ice water and made alkaline with 20% sodium hydroxide. The precipitate formed was filtered off and purified by recrystalization from toluene. The title compound was obtained as white crystals. Yield 7.2 parts (87.2%), m.p. 244-246° C.

Elemental analysis of the title compound gave the following results:

| Percent | : C | H | N |
|---|---|---|---|
| Calcd. for $C_{26}H_{27}N_3O_2$ | : 75.51 | 6.59 | 10.16 |
| Found | : 75.39 | 6.61 | 10.00 |

Examples 2 to 18

Further compounds of the formula I were made by substituting the corresponding primary amino-substituted phthalide (furopyridine or furopyrazine) for the 3-(2-amino-4-dimethylaminophenyl)-3-(4-dimethylaminophenyl)-6-dimethylaminophthalide used in Example 1. These compounds and that of Example 1 are set out in Table 1 below.

Example 19

Preparation of Heat-Sensitive Recording Paper

4 parts of the fluorene-spiro-phthalide of Example 1 were milled with 10 parts of a 10% aqueous solution of polyvinyl alcohol and 6 parts of water to give Dispersion A. Separately, 4 parts of bisphenol A were milled with 10 parts of a 10% aqueous solution of polyvinyl alcohol and 6 parts of water to give Dispersion B.

1 part of Dispersion A and 6 parts of Dispersion B were mixed, and the resulting mixture was coated onto a paper sheet support and dried to give heat-sensitive recording paper. When heat was applied by a heat pen to this heat-sensitive recording paper a green coloured image was produced. This coloured image has excellent light-fastness and had a marked absorption in the near ingfrared region. When imaged in OCR

format the characters could be read by an infrared OCR reader.

Example 20

Preparation of Pressure-Sensitive Copy Paper

The fluorene-spiro-phthalide Example 1 was dissolved in an alkylnaphthalene solvent, and microencapsulated by a conventional method. The microcapsules were coated onto the surface of a paper sheet support, and dried to give an upper sheet (CB sheet). Correspondingly, a solid phenolic resin was coated onto the surface of a paper sheet support to give a lower sheet (CF sheet). When the CB sheet and the CF sheet were superposed so that both the coated surfaces faced each other, writing pressure on the upper surface of the CB sheet e.g. with a pen, gave a bluish-green copied image on the lower sheet. This copied image is fairly lightfast showing absorption in the near infrared region. When imaged in OCR format the characters could be read by an infrared OCR reader.

Example 21

A coloured image was formed by reacting compounds of the invention with Bisphenol A ( 2,2-bis(4'-hydroxyphenyl) propane). The compounds of Examples 1 to 17 each gave a green visible image and the compound of Example 18 gave a brown visible image. All the images also showed a substantial absorption in the near infrared which could be detected by OCR devices.

Table 1

| Ex. No. | E | $R_1$ | $R_2$ | $R_3$ | $R_4$ | $R_5$ | $R_6$ | M. Pt. (°C) |
|---|---|---|---|---|---|---|---|---|
| 1 | II | $-CH_3$ | $-CH_3$ | $-CH_3$ | $-CH_3$ | $-CH_3$ | $-CH_3$ | 244-6 |
| 2 | II | $-C_2H_5$ | $-C_2H_5$ | $-CH_3$ | $-CH_3$ | $-CH_3$ | $-CH_3$ | 235-8 |
| 3 | II | $-C_2H_5$ | $-C_2H_5$ | $-C_2H_5$ | $-C_2H_5$ | $-CH_3$ | $-CH_3$ | 188-91 |
| 4 | II | $-C_4H_9$ | $-C_4H_9$ | $-CH_3$ | $-CH_3$ | $-CH_3$ | $-CH_3$ | 150-1 |
| 5 | II | $-C_4H_9$ | $-C_4H_9$ | $-C_2H_5$ | $-C_2H_5$ | $-CH_3$ | $-CH_3$ | 139-41 |
| 6 | II | $-CH_3$ | $-CH_3$ | $-CH_3$ | $-CH_3$ | $-C_2H_5$ | $-C_2H_5$ | 244-6 |
| 7 | II | $-C_2H_5$ | $-C_2H_5$ | $-CH_3$ | $-CH_3$ | $-C_2H_5$ | $-C_2H_5$ | 164-6 |
| 8 | II | $-C_4H_9$ | $-C_4H_9$ | $-CH_3$ | $-CH_3$ | $-C_2H_5$ | $-C_2H_5$ | 128-31 |
| 9 | II | $-C_2H_5$ | $-C_2H_5$ | $-C_2H_5$ | $-C_2H_5$ | $-C_2H_5$ | $-C_2H5$ | 210-11 |
| 10 | II | $-C_4H_9$ | $-C_4H_9$ | $-C_2H_5$ | $-C_2H_5$ | $-C_2H_5$ | $-C_2H_5$ | 148-50 |
| 11 | II | $-CH_3$ | $-CH_3$ | $-CH_3$ | $-CH_3$ | $-C_4H_9$ | $-C_4H_9$ | 197-8 |
| 12 | II | $-C_2H_5$ | $-C_2H_5$ | $-CH_3$ | $-CH_3$ | $-C_4H_9$ | $-C_4H_9$ | 177-8 |
| 13 | II | $-CH_3$ | $-CH_3$ | $-CH_3$ | $-H$ | $-CH_3$ | $-CH_3$ | 259-61 |
| 14 | II | $-CH_3$ | $-CH_3$ | $-CH_2C_6H_5$ | $-CH_2C_6H_5$ | $-CH_3$ | $-CH_3$ | 238-40 |
| 15 | II | $-CH_3$ | $-CH_3$ | $-(CH_2)_4-$ | | $-CH_3$ | $-CH_3$ | 278.5-9.5 |
| 16 | II | $-CH_3$ | $-CH_3$ | $-(CH_2)_5-$ | | $-CH_3$ | $-CH_3$ | 229.5-31 |
| 17 | II | $-CH_3$ | $-CH_3$ | $-CH_3$ | 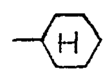 | $-CH_3$ | $-CH_3$ | 250-2 |
| 18 | III | $-C_2H_5$ | $-C_2H_5$ | $-C_2H_5$ | $-C_2H_5$ | - | - | 228-9.5 |

## Claims

**1.** A compound of the general formula:

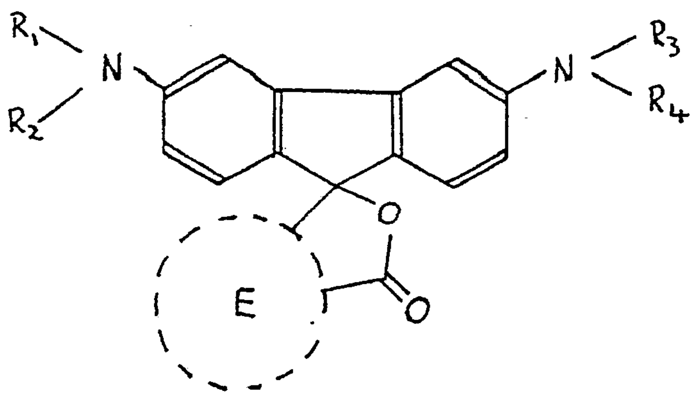

(I)

where E is a group of one of the formulae:

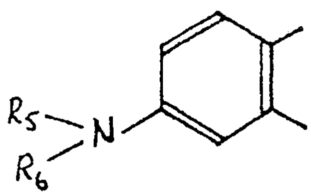 (II), 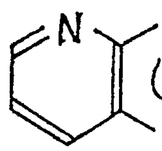 (III) or 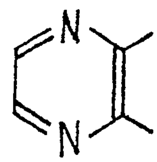 (IV)

$R_1$, $R_2$, $R_3$, $R_4$, $R_5$ and $R_6$ are each independently a hydrogen atom, a $C_1$ to $C_{12}$ alkyl group, a cycloalkyl group or a phenyl or benzyl group optionally substituted with one or more $C_1$ to $C_5$ alkyl groups or $R_1$ and $R_2$ $R_3$ and $R_4$ and/or $R_5$ and $R_6$, together with the nitrogen atom to which they are attached represent a 5- or 6- membered heterocyclic ring group.

2. A compound as claimed in Claim 1 characterized in that any of $R_1$, $R_2$, $R_3$, $R_4$, $R_5$ and $R_6$ is a $C_1$ to $C_8$ alkyl group.

3. A compound as claimed in Claim 1 characterized in that E is a group of the formula II and all of $R_1$, $R_2$, $R_3$, $R_4$, $R_5$ and $R_6$ are independently $C_1$ to $C_4$ alkyl groups.

4. A compound as claimed in Claim 1 characterised in that any of $R_1$ and $R_2$, $R_3$ and $R_4$ and/or $R_5$ and $R_6$ together with the nitrogen atom to which they are attached represent a pyrrolidino, a piperidino, a morpholino, a thiomorpholino or a piperazine group.

5. A method of making a compound as claimed in Claim 1 which comprises diazotizing a compound of the general formula:

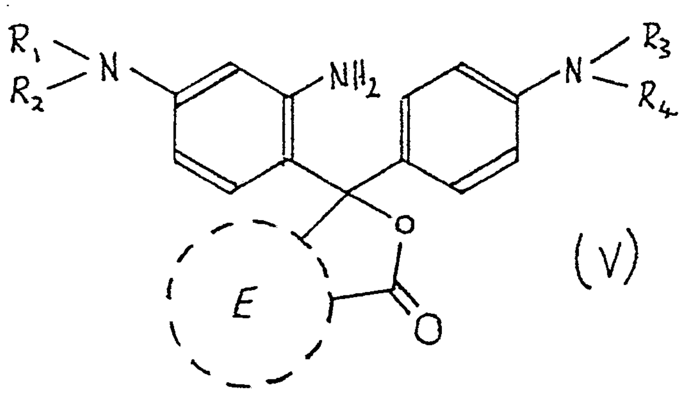

(V)

where $R_1$, $R_2$, $R_3$, $R_4$ and E are as defined in Claim 1, and thereafter effecting ring closure.

6. A method as claimed in Claim 5 characterized in that E is a group of the formula II and all of $R_1$, $R_2$, $R_3$, $R_4$, $R_5$ and $R_6$ are independently $C_1$ to $C_4$ alkyl groups.

7. A method as claimed in Claim 5 characterized in that the diazotization of the precursor amino substituted phthalide, furopyridine or furopyrazine is carried out in solution in sulphuric acid at a temperature of from -5 to 10 °C and that the ring closing reaction is carried out at a temperature of from 10 to 100 °C.

8. A method as claimed in any one of Claims 5 to 7 characterized in that the ring closing reaction is carried out in the presence of elemental copper or a copper compound.

9. Record material incorporating, as a colour former, at least one chromogenic compound as claimed in any one of claims 1 to 4.

10. Record material as claimed in Claim 9 in the form of a manifold set of pressure sensitive record material which comprises an upper sheet carrying on the lower surface thereof a coating of a microencapsulated solution of a chromogenic composition including at least one compound as claimed in any one of claims 1 to 4 a lower sheet carrying on the upper surface thereof a coating comprising an electron accepting (acidic) co-reactant and, optionally, one or more intermediate sheets each of which has on its upper surface a coating comprising an electron accepting (acidic) co-reactant and on its lower surface a coating comprising a microencapsulated solution of a chromogenic composition, including at least one compound of this invention.

11. Record material as claimed in Claim 9 in the form of heat sensitive record material which comprises a sheet, especially a paper sheet, carrying a coating comprising finely divided solid particles of a chromogenic compound as claimed in any one of claims 1 to 4 and finely divided solid particles of an acidic co-reactant dispersed in a thermographically acceptable binder.

**Revendications**

1. Composé de formule générale :

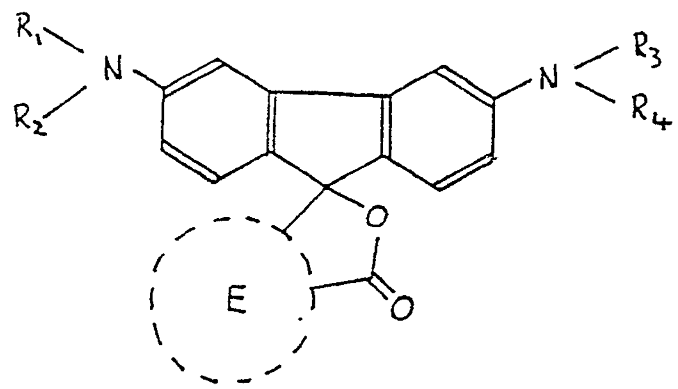

(I)

où E est un groupe d'une des formules :

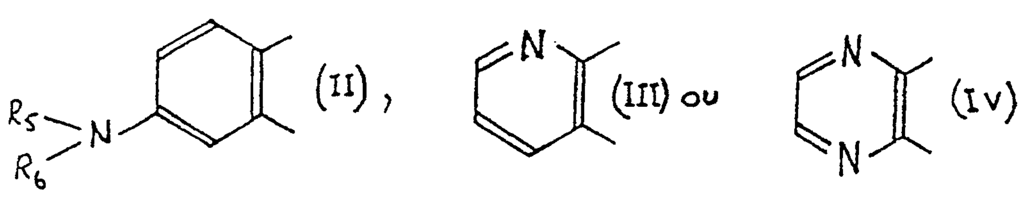

(II), (III) ou (IV)

$R_1$, $R_2$, $R_3$, $R_4$, $R_5$, et $R_6$ représentent indépendamment un atome d'hydrogène, un groupe alkyle $C_1$ à $C_{12}$, un groupe cycloalkyle ou un groupe benzyle ou phényle éventuellement substitué par un ou

plusieurs groupes alkyle $C_1$ à $C_6$, ou $R_1$ et $R_2$, $R_3$ et $R_4$ et/ou $R_5$ et $R_6$, avec l'atome d'azote auquel ils sont rattachés, représentent un groupement hétérocyclique à 5 ou 6 chaînons.

2. Composé tel que revendiqué dans la revendication 1, caractérisé en ce que l'un quelconque de $R_1$, $R_2$, $R_3$, $R_4$, $R_5$, et $R_6$ représente un groupe alkyle $C_1$ à $C_8$.

3. Composé tel que revendiqué dans la revendication 1, caractérisé en ce que E est un groupe de formule II et l'ensemble des substituants $R_1$, $R_2$, $R_3$, $R_4$, $R_5$ et $R_6$, représente indépendamment un groupe alkyle $C_1$ à $C_4$.

4. Composé tel que revendiqué dans la revendication 1, caractérisé en ce que l'un quelconque de $R_1$ et $R_2$, $R_3$ et $R_4$ et/ou $R_5$ et $R_6$, avec l'atome d'azote auquel ils sont rattachés, représente un groupe pyrrolidino, pipéridino, morpholino, thiomorpholino ou pipérazine.

5. Procédé de préparation d'un composé tel que revendiqué dans la revendication 1, comprenant les étapes consistant à soumettre à une réaction de diazotation un composé de formule générale :

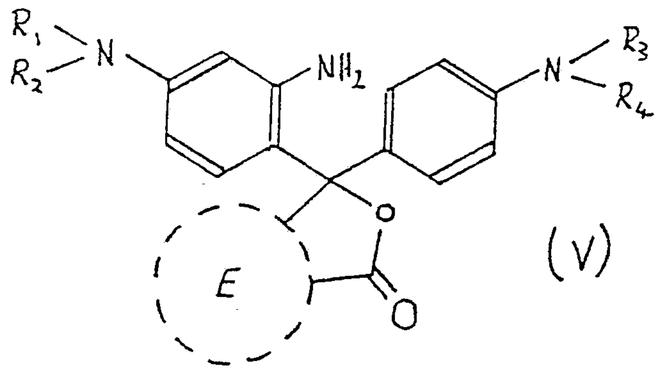

(V)

où $R_1$, $R_2$, $R_3$, $R_4$ et E sont tels que définis dans la revendication 1, et à effectuer ensuite une réaction de cyclisation.

6. Procédé tel que revendiqué dans la revendication 5, caractérisé en ce que E est un groupe de formule II et l'ensemble des substituants $R_1$, $R_2$, $R_3$, $R_4$, $R_5$ et $R_6$ représente indépendamment des groupes alkyle $C_1$ à $C_4$.

7. Procédé tel que revendiqué dans la revendication 5, caractérisé en ce que la diazotation du précurseur phthalide, furopyridine ou furopyrazine amino substitué est effectuée en solution dans de l'acide sulfurique à une température allant de -5 à 10 °C, et que la réaction de cyclisation est effectuée à une température allant de 10 à 100 °C.

8. Procédé tel que revendiqué dans l'une quelconque des revendications 5 à 7, caractérisé en ce que la réaction de cyclisation est effectuée en présence de cuivre élémentaire ou d'un composé cuivré.

9. Matériau d'enregistrement comprenant, en tant que formateur de couleurs, au moins un composé chromogène tel que revendiqué dans l'une quelconque des revendications 1 à 4.

10. Matériau d'enregistrement tel que revendiqué dans la revendication 9, sous la forme d'un ensemble de matériaux d'enregistrement sensibles à la pression, lequel comprend une feuille supérieure portant sur sa face inférieure un revêtement d'une solution microencapsulée d'une composition chromogène comprenant au moins un composé tel que revendiqué dans l'une quelconque des revendications 1 à 4, une feuille inférieure portant sur sa face supérieure un revêtement comprenant un partenaire réactionnel accepteur d'électrons (acide) et, facultativement, une ou plusieurs feuilles intermédiaires dont chacune a sur sa face supérieure un revêtement comprenant un partenaire réactionnel accepteur d'électrons (acide) et sur sa face inférieure un revêtement comprenant une solution microencapsulée d'une composition chromogène, comprenant au moins un composé de la présente invention.

11. Matériau d'enregistrement tel que revendiqué dans la revendication 9 sous la forme d'un matériau d'enregistrement sensible à la chaleur, lequel comprend une feuille, notamment une feuille de papier, portant un revêtement comprenant des particules solides finement divisées d'un composé chromogène tel que revendiqué dans l'une quelconque des revendications 1 à 4 et des particules solides finement divisées d'un partenaire réactionnel acide dispersé dans un liant adapté à la thermographie.

**Patentansprüche**

1. Verbindung der allgemeinen Formel:

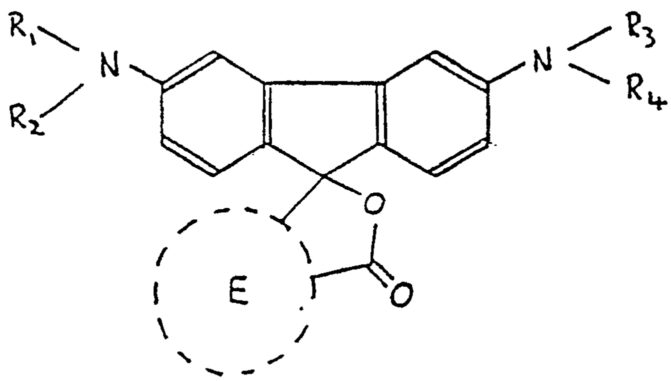

wo E eine Gruppe von einer der Formeln ist:

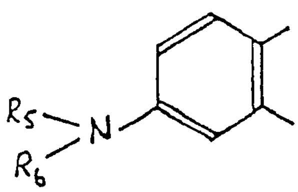

(II),

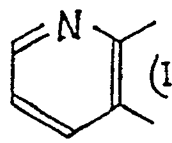

(III) oder

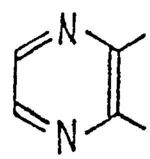

(IV)

$R_1$, $R_2$, $R_3$, $R_4$, $R_5$ und $R_6$ jeweils unabhängig ein Wasserstoffatom, eine $C_1$ bis $C_{12}$ Alkylgruppe, eine Cycloalkylgruppe oder eine Phenyl- oder Benzylgruppe, gegebenenfalls mit einer oder mehreren $C_1$ bis $C_5$ Alkylgruppen substituiert, sind oder $R_1$ und $R_2$, $R_3$ und $R_4$ und/oder $R_5$ und $R_6$ zusammen mit dem Stickstoffatom, an das sie gebunden sind, eine 5- oder 6-gliedrige heterocyclische Ringgruppe darstellen.

2. Verbindung nach Anspruch 1, **dadurch gekennzeichnet**, daß jeder von $R_1$, $R_2$, $R_3$, $R_4$, $R_5$ und $R_6$ eine $C_1$ bis $C_8$ Alkylgruppe ist.

3. Verbindung nach Anspruch 1, **dadurch gekennzeichnet**, daß E eine Gruppe der Formel II ist und $R_1$, $R_2$, $R_3$, $R_4$, $R_5$ und $R_6$ jeweils unabhängig $C_1$ bis $C_4$ Alkylgruppen sind.

4. Verbindung nach Anspruch 1, **dadurch gekennzeichnet**, daß $R_1$ und $R_2$, $R_3$ und $R_4$ und/oder $R_5$ und $R_6$ zusammen mit dem Stickstoffatom, an das sie gebunden sind, eine Pyrrolidin-, eine Piperidin-, eine Morpholin-, eine Thiomorpholin- oder eine Piperazingruppe darstellen.

5. Verfahren zur Herstellung einer Verbindung nach Anspruch 1, umfassend das Diazotieren einer Verbindung der allgemeinen Formel:

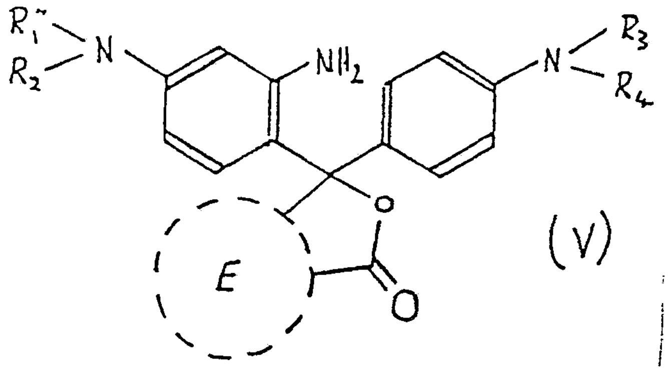

(V)

worin $R_1$, $R_2$, $R_3$, $R_4$ und E wie in Anspruch 1 definiert sind, und danach das Bewirken eines Ringschlusses.

6. Verfahren nach Anspruch 5, **dadurch gekennzeichnet**, daß E eine Gruppe der Formel II ist und $R_1$, $R_2$, $R_3$, $R_4$, $R_5$ und $R_6$ jeweils unabhängig $C_1$ bis $C_4$ Alkylgruppen sind.

7. Verfahren nach Anspruch 5, **dadurch gekennzeichnet**, daß die Diazotierung der aminsubstituierten Phthalid-, Furopyridin- oder Furopyrazinvorstufe in Lösung in Schwefelsäure bei einer Temperatur von -5 bis 10 °C durchgeführt wird und daß die Ringschlußreaktion bei einer Temperatur von 10 bis 100 °C durchgeführt wird.

8. Verfahren nach einem der Ansprüche 5 bis 7, **dadurch gekennzeichnet**, daß die Ringschlußreaktion in Gegenwart von elementarem Kupfer oder einer Kupferverbindung durchgeführt wird.

9. Aufzeichnungsmaterial, das als Farbbildner mindestens eine chromogene Verbindung nach einem der Ansprüche 1 bis 4 enthält.

10. Aufzeichnungsmaterial nach Anspruch 9 in Form eines mehrteiligen Satzes von drucksensitivem Aufzeichnungsmaterial, umfassend ein oberes Blatt, das auf seiner unteren Oberfläche eine Beschichtung einer mikroverkapselten Lösung einer chromogenen Zusammensetzung trägt, die mindestens eine Verbindung nach einem der Ansprüche 1 bis 4 enthält, ein unteres Blatt, das auf seiner oberen Oberfläche eine Beschichtung trägt, die einen elektronenaufnehmenden (sauren) Coreaktanden aufweist und gegebenenfalls ein oder mehrere Zwischenblätter, von denen jedes auf seiner oberen Oberfläche eine Beschichtung, die einen elektronenaufnehmenden (sauren) Coreaktanden enthält, und auf seiner unteren Oberfläche eine Beschichtung aufweist, die eine mikroverkapselte Lösung einer chromogenen Zusammensetzung enthält, die mindestens eine erfindungsgemäße Verbindung beinhaltet.

11. Aufzeichnungsmaterial nach Anspruch 9 in Form eines hitzesensitiven Aufzeichnungsmaterials, das ein Blatt, insbesondere ein Papierblatt enthält, das eine Beschichtung trägt, die fein verteilte feste Teilchen einer chromogenen Verbindung nach einem der Ansprüche 1 bis 4 und fein verteilte Teilchen eines sauren Coreaktanden, dispergiert in einem thermographisch brauchbaren Bindemittel, enthält.